# EUROPEAN PATENT APPLICATION

(11) **EP 4 141 906 A1**
(43) Date of publication of application: **01.03.2023**
(21) Application number: 21821017.7
(22) Date of filing: 28.04.2021
(51) Int. Cl.: H01J 37/20, G01N 23/2202, G01N 23/2204, G01N 23/2251, G01N 1/28

(54) **OBSERVATION DEVICE FOR GAS UNDER OBSERVATION, METHOD OF OBSERVING IONS UNDER OBSERVATION, AND SAMPLE HOLDER**

(30) Priority: 09.06.2020 JP 2020100461
(71) Applicant: National Institute for Materials Science, Tsukuba-shi, Ibaraki 305-0047 (JP)
(72) Inventor: NAKAMURA, Akiko, Tsukuba-shi, Ibaraki 305-0047 (JP); MURASE, Yoshiharu, Tsukuba-shi, Ibaraki 305-0047 (JP); MIYAUCHI, Naoya, Tsukuba-shi, Ibaraki 305-0047 (JP); YAKABE, Taro, Tsukuba-shi, Ibaraki 305-0047 (JP)
(74) Representative: Isarpatent
(86) International application number: PCT/JP2021/017111
(87) International publication number: WO 2021/251026

(57) **Abstract**

Provided are an observation device for observation target gas capable of independently moving a sample holder, to which a sample in a thin plate form is mounted, within an analysis chamber, an observation method, and a sample holder that can be used suitably. This observation device comprises: a scanning electron microscope 15 for detecting secondary electrons generated by irradiating the sample 17 with an electron beam within the analysis chamber 11; a sample holder 12 having a cell 12c for housing the observation target gas, an open window W of the cell 12c, and a sample mounting part 12b to which the sample 17 can be mounted so as to block the open window W; and an observation target ion detecting unit 20 for irradiating the front surface of the sample 17 with an electron beam in a state where the observation target gas in the cell 12c contacts the back surface of the sample 17 and detecting observation target ions derived from the observation target gas generated by the electron beam. In a state where the observation target gas is housed in the cell and the sample 17 is mounted to the sample mounting part 12b of the sample holder, the entire hydrogen cell 12c can be sealed.

## Description

### Technical Field

The present invention relates to a device for observing target gas capable of exciting an observation target gas, such as hydrogen desorbed from a solid sample, by an electron beam of an electron microscope and creating an image of the region on the surface of the solid sample where ions derived from the observation target gas and detached from the surface of the solid sample are present, a method of observing ions under observation, and a sample holder.

### Background Art

Electron stimulated desorption (hereinafter called as ESD method), which is a known process in the field of surface analysis, is a method of analyzing the surface of a solid by ionizing and desorbing atoms having attached to the solid sample by electron irradiation. The ESD method makes it possible to directly observe the observation target gas such as hydrogen desorbed from the solid sample in the real time (Non-Patent Literatures 1 and 2).

Taking hydrogen as an example of the observation target gas, it becomes possible, by using the ESD method, to visualize the positional information of the hydrogen present on the surface of the solid sample. However, since the measurement cannot be continued once the hydrogen has been desorbed completely, this method is unsuitable for the measurement of amounts of discharged hydrogen existing as trace in the steel.

The inventors et. al have developed a device for observing hydrogen permeation and diffusion path, which includes a collecting mechanism with high hydrogen ion yield effect and an ion energy decomposing unit for selectively allowing hydrogen ions to permeate, applicable when hydrogen atoms that diffuse within the sample and permeate (desorb) the surface side are acquired by the ESD method by introducing hydrogen to the sample from its back side, and a method of measuring hydrogen ions that permeate the sample by using the observation device (Paten Literatures 1 and 2).

The above-mentioned hydrogen permeation position detecting device, which excites and detaches the hydrogen desorbed from the sample by using scanning electrons of the electron microscope to create its image, is a type of operando hydrogen microscope. The operando hydrogen microscope is an observation device that makes hydrogen permeate a material and obtains a two-dimensional image of the discharge part of the hydrogen.

With the method of visualizing permeated hydrogen using the operando hydrogen microscope of prior art as described in Patent Literatures 1 and 2, it is possible to observe hydrogen permeating a sample nondestructively and in real time by placing the sample within a vacuum analysis chamber as a diaphragm while being supported by a sample holder, and by supplying hydrogen from a hydrogen pipe to the back side of the sample.

### Citation List

### Patent Literature

Patent Literature 1: JP 2017-187457 A
Patent Literature 2: JP 2019-145255 A

### Non-Patent Literature

Non-patent Literature 1: Akiko N. Itakura, Yoshiharu Murase, Masahiro Tosa, Shinji Suzuki, Shoji Takagi, Tetsuji Gotoh, "Effect of Surface Processing on Hydrogen Desorption from Stainless Steel to UHV," J. Vac. Soc. Jpn., Vol. 57, No. 1, pp. 23-26, 2014
Non-patent Literature 2: Naoya Miyauchi, Shinji Suzuki, Shoji Takagi, Tetsuji Gotoh, Yoshiharu Murase, Akiko N. Itakura, "Electron Stimulated Desorption Measurement of Permeated Hydrogen through Stainless Steel Membrane," J. Vac. Soc. Jpn., Vol. 58, No. 10, pp. 31-35, 2015

### Summary of Invention

### Problem to be solved by the Invention

By the way, to the hydrogen permeation position detecting device disclosed in Patent Literatures 1 and 2, a gas supply unit is provided to the back side of the sample in order to supply the observation target gas such as hydrogen. The sample is supported as a diaphragm by the sample holder, and the observation target gas is supplied to the back side in a state where the front surface side of the sample is arranged within the analysis chamber in ultra-high vacuum state. Consequently, the hydrogen gas supply unit must supply the observation target gas supplied from an external supply source to the back side of the sample supported by the sample holder within the analysis chamber. To do so, it is necessary to provide an introduction line and connect it to the sample holder so as to prevent the observation target gas from moving into the analysis chamber.

With the above-mentioned device, the sample holder and the sample supported by the sample holder cannot be moved independently of each other within the analysis chamber due to the observation target gas introduction line, and the observation operation is inhibited significantly. For example, the sample cannot be observed while it is rotated. Also for example, two or more measurement means cannot be combined in microscopic structural analysis. That is why further improvement has been desired in the capabilities to study the behavior of various observation target gases, including the coefficient of diffusion of observation target gas within the sample.

In view of such circumstances, the purpose of the present invention is to provide an observation device and a method of observing the observation target ions where a sample holder, to which a solid sample (hereinafter simply referred to as sample) such as a metal material and a semiconductor material is mounted, can be moved independently within the analysis chamber to decrease the limitation in the observation operation and improve the performance to study the behavior of various observation target gases. Another objective of the present invention is to provide a sample holder that can suitably be used for such device for observing target gases.

### Means for Solving the Problem

To achieve the above objective, a target gas observation device of the present invention comprises: a scanning electron microscope for detecting secondary electrons generated by emitting an electron beam to a sample within an analysis chamber; a sample holder having a cell for housing the observation target gas as well as an open window of the cell and a sample mounting part to which the sample can be attached in a state blocking the open window; and an observation target ion detecting unit for detecting observation target ions derived from the observation target gas generated by the electron beam after emitting the electron beam to the front surface of the sample in a state where the observation target gas contacts the back surface of the sample, wherein the entire cell can be tightly sealed in a state where the observation target gas is housed in the cell and the sample is mounted to the sample mounting part of the sample holder.

The target gas observation device of the present invention can house an absorbing material for absorbing the observation target gas in the cell. To the sample mounting part of the device, a window frame area, against which the sample can be appressed, is provided around the open window. The window frame area comprises: an inner seal surrounding the open window; an outer seal surrounding the inner seal; an exhaust port for exhausting the area between the inner seal and the outer seal ; and a valve for opening and closing the exhaust port. It is preferable that the sample holder has an exhaust port and introduction path for exhausting the cell and introducing the observation target gas and a valve for opening and closing the exhaust port and introduction path at positions different from the open window. The exhaust port and the introduction path may be installed in a state attachable to detachable from the sample holder. The sample holder may be attached to a sample stage in a state removable to outside the analysis chamber. The sample stage may be installed within the analysis chamber in an insertable and removable state. The sample stage may be comprised: a rotating mechanism; a temperature control; and an ion focusing mechanism, and the sample stage is configured to heat the sample.

With a method of observing the observation target ions of the present invention to achieve the above objective, to measure the observation target ions by using the above-mentioned target gas observation device, the method of observing target ions comprising the steps of: mounting the sample to the sample mounting part to block the open window; housing the observation target gas in the cell; placing the entire sample holder within the analysis chamber with the entire cell sealed; and detecting the the observation target ions generated by emitting the electron beam to the front surface of the sample in a state where the observation target gas contacts the back surface of the sample.

With the method of observing the observation target ions according to the present invention, it is desirable that the observation target ions be detected by precisely changing the position of the sample holder within the analysis chamber. It is desirable that a material for absorbing the observation target gas and the observation target gas are housed in the cell. Furthermore, it is also desirable that mounting the sample to the sample mounting part in a state where the sample is appressed against a window frame area surrounding the open window; blocking the open window; exhausting the cell and introducing the observation target gas to the cell at a position different from the open window; and placing the entire sample holder within the analysis chamber after the entire cell is sealed.

To achieve the above objective, the sample holder of the present invention comprises: a holder main body that can be housed within an analysis chamber of a scanning electron microscope for detecting secondary electrons generated by emitting an electron beam; a cell for housing an observation target gas provided within the holder; a sample mounting part to which the sample can be mounted; and an open window of the cell provided at the sample mounting part, wherein by mounting the sample to the sample mounting part, blocking the open window , the cell is sealed in a state where the observation target gas contacts the back surface of the sample.

### Effects of Invention

According to the device for observing target gas and the method of observing target ions, the gas under observation is housed in a cell and the entire cell is sealed off with the sample attached to the sample mounting part. Therefore, if the observation target gas is housed in the cell and the cell is sealed in advance, it is possible, when detecting the observation target ions derived from the observation target gas, to make the observation target gas housed in the cell contact the back surface of the sample, and thus it is unnecessary to supply the observation target gas from outside of the analysis chamber to the cell. It is therefore possible to eliminate the introduction line of observation target gas to the analysis chamber during observation operation.

According to the present invention, a highly sophisticated observation atmosphere surrounding the sample within the analysis chamber and the sample holder can be maintained without fail. At the same time, the operation of the sample holder within the analysis chamber is not inhibited by the introduction line of the observation target gas. According to the present invention, the sample holder to which the sample is integrally mounted can be moved independently within the analysis chamber. For example, the sample can be observed while being rotated. It is therefore facilitated to combine two or more measurement means for microscopic structural analysis, etc., which improves the performance to study various observation target gas behaviors, including finding the coefficient of diffusion of the observation target gas within the sample.

### Brief Description of Drawings

FIG. 1 is a view schematically showing a type of device for observing the permeation and diffusion path of observation target gas in the embodiment of the present invention.
FIG. 2 is a cross-sectional view schematically showing the sample holder of this embodiment.
FIG. 3 is a block diagram showing the structure of a control unit in this embodiment.
FIG. 4 is a block diagram showing the structure of a electron stimulated desorption overall control unit.
FIG. 5 is a schematic view showing the relation between the electron source scanning and the two-dimensional measurement of an ESD image.
FIG. 6 is a flow chart for measuring a two-dimensional ESD image by electron beam scanning.
FIG. 7 is a flow chart describing the process of fastening a sample to the sample holder in this embodiment.
FIG. 8 is a flow chart describing the process of introducing an observation target gas to the sample holder in this embodiment.

### Embodiments of the Invention

The embodiment of the present invention will hereinafter be described in detail by referring to drawings.

In the following description, hydrogen is taken as an example of observation target gas, but the target gas of the present invention is not limited to hydrogen. The observation target gas may be molecules or ions of deuterium, helium, oxygen, nitrogen, water or any one of gases related to the manufacture of the sample or the purpose of use of the sample, namely observation target ions derived from the observation target gas, or molecules or ions derived from a plurality of such gases. In this embodiment, deuterium is used in the following description to easily distinguish it from the hydrogen gas remaining as background. The sample 17 under observation is not particularly limited, but in this embodiment, a plate-like sample made of various metals, etc. is taken as an example.

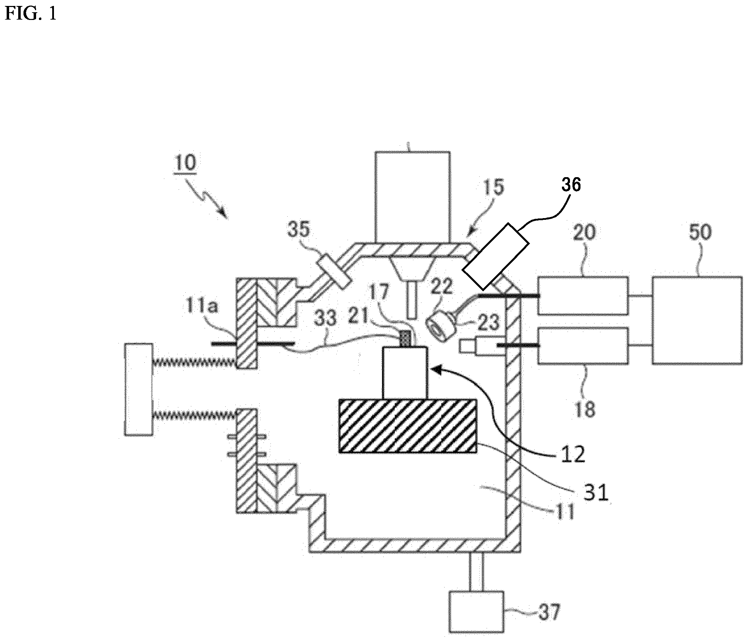

In this embodiment, a hydrogen permeation and diffusion path observation device, which is a type of observation target gas permeation and diffusion path observation device, is described. FIG. 1 schematically shows the structure of the hydrogen permeation and diffusion path observation device 10 according to the embodiment.

The hydrogen permeation and diffusion path observation device 10 has a scanning electron microscope 15. This scanning electron microscope 15 is provided with an analysis chamber 11 that houses a sample 17 together with an electron source 16 for emitting an electron beam to the sample 17, and a secondary electron detector 18 that is disposed in the analysis chamber 11 for detecting secondary electrons generated from the electron beam emitted to the sample 17.

The hydrogen permeation and diffusion path observation device 10 comprises: a sample holder 12 housing hydrogen to which the sample 17 is mounted; a sample stage 31 to which the sample holder 12 is mounted; an electron source 16 for emitting an electron beam to the front surface of the sample 17 in a state where hydrogen gas contacts the back surface of the sample 17, a hydrogen ion detecting unit 20 for detecting hydrogen ions derived from the hydrogen gas generated by the electron beam emitted from the electron source 16; a sample temperature measuring section 33 for measuring the temperature of the sample 17; a sample position adjusting unit (not shown) for adjusting the position of the sample 17; an evacuation unit 37; and a control unit 50.

The sample stage 31 has a structure allowing the sample holder 12 to be attached or detached. The sample stage 31 may be fastened within the analysis chamber 11, but in this embodiment, it is provided in a state where it can be taken in and out of the analysis chamber 11.

The sample stage 31 is capable of heating the sample 17 to a level higher than that of the room temperatures so as to promote diffusion of hydrogen, and has a halogen lamp capable of heating the sample 17 and the sample holder 12, for example. The temperature of the sample 17 to be heated is measured by the sample temperature measuring section 33 as shown in FIG. 1. The sample stage 31 supports the sample holder 12, and is structured to ensure the precise movement of the sample holder 12 against the electron source 16 or the hydrogen ion detecting unit 20. The sample stage 31 in this embodiment also combines features of movement in x, y, and z directions, angle rotation, temperature control, and ion focusing mechanism.

The evacuation unit 37 includes a vacuum pump (not shown) such as a turbo molecular pump, gate valve, vacuum gauge, etc. to bring the analysis chamber 11 to be in an ultrahigh vacuum state as the observation atmosphere. The evacuation unit 37 evacuates the analysis chamber 11 to a degree of vacuum allowing a SEM image to be obtained, 1.0 × 10⁻⁷ Pa or lower, for example.

To the analysis chamber 11, a mass analyzer 35 for analyzing elements remaining within the analysis chamber 11 may be provided. The mass analyzer 35 is a quadruple mass analyzer, for example. To the analysis chamber 11, an auger electron spectroscopy analyzer 36 may also be provided. The auger electron spectroscopy analyzer 36 measures the amount of carbon, etc. present on the surface of the sample 17. It is also allowed to remove the background present on the surface of the sample 17 such as hydrogen and carbon by using the sputtering source provided within the analysis chamber 11 or by irradiating an electron beam before obtaining the ESD image to be described later.

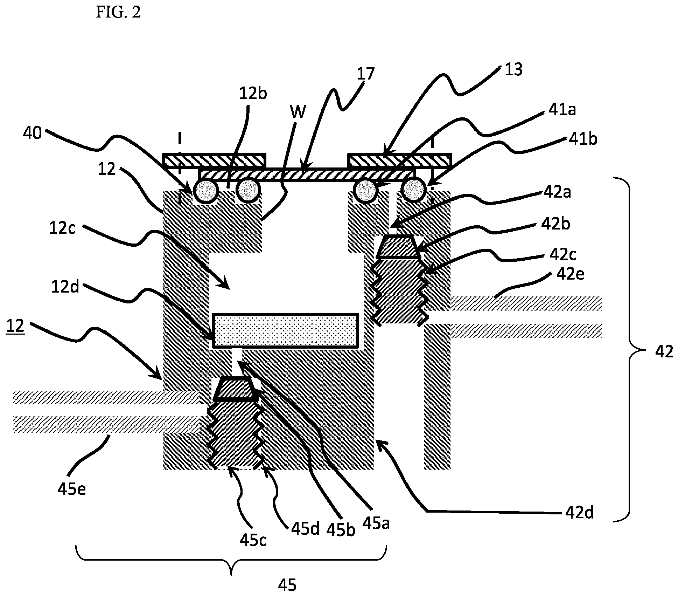

FIG. 2 is a cross-sectional view schematically showing the sample holder 12 in this embodiment. The sample holder 12 comprises: a holder main body 12a, which is made smaller than the analysis chamber 11 so that the entire body can be housed within the analysis chamber 11; a hydrogen cell 12c provided within the holder main body 12a for housing hydrogen gas, a sample mounting part 12b to which the sample 17 is mounted; an open window W of the hydrogen cell 12c provided at the sample mounting part 12b; a sealing part 40 that is appressed against the back surface of the sample 17 mounted to the sample mounting part 12b to seal the open window W; and a hydrogen gas introduction unit 45 for evacuating the hydrogen cell 12c and introducing hydrogen gas as the observation target gas.

Since the holder main body 12a is required to suppress the amount of release of hydrogen gas, it is made of an ultrahigh vacuum material such as stainless steel, copper, glass, and Teflon (registered trademark). If the heating is necessary, materials that can be baked at the temperature of approximately 120°C are used, for example. The sample mounting part 12b can be made of copper to ensure higher heat conduction. The entire holder main body 12a is formed in a size capable of being housed within the analysis chamber 11. The holder main body 12a may also be formed in an integrated massive form, but also may have an opening and closing unit for opening and closing the hydrogen cell 12c provided inside. To outside the holder main body 12a, a mounting structure that can be attached to the sample stage 31 may be provided.

The hydrogen cell 12c is a small chamber for sealing hydrogen gas by ensuring high airtightness inside the holder main body 12a, and its shape is not particularly limited. To its top, an open window W is provided. In this embodiment, fine powder particles of hydrogen absorbing alloy 12d are housed in the hydrogen cell 12c as an absorbing material.

The sample mounting part 12b is the mounting area for the sample 17 formed at the upper part of the holder main body 12a, and has the open window W of the hydrogen cell 12c. When the sample 17 is mounted, this open window W is completely blocked by the back surface of the sample. To the sample mounting part 12b, a window frame area against which the sample 17 abut is provided surrounding the periphery of the open window W, and the sealing part 40 is provided in the window frame area. The sample 17 is mounted to the sample mounting part 12b in a state blocking the open window W.

The sealing part 40 makes the outer periphery of the sample 17 abut against the window frame area around the open window W to seal the periphery. Various vacuum sealing methods are adopted and a metal O-ring, a metal thin line seal, an elastomer O-ring, etc. can be adopted. When the elastomer seal is used for example, the sealing is performed as follows to adopt to the ultrahigh vacuum environment. The sealing part 40 in this embodiment has the elastomer seal (inside) 41a as the seal consecutively surrounding the open window W in circular state, the elastomer seal (outside) 41b as the seal consecutively surrounding the elastomer seal 41a in circular state, and an exhaust unit 42 for exhausting the space between the elastomer seal 41a and the elastomer seal 41b.

It is preferable that the inside elastomer seal 41a and the outside elastomer seal 41b have the elasticity or flexibility sufficient to exhaust the space between the elastomer seal 41a and the elastomer seal 41b.

The exhaust unit 42 comprises: a differential exhaust port 42a that is open so as to be directly connected to the space between the elastomer seals 41a and 41b; a stem chip 42b as an opening and closing valve to open and close the differential exhaust port 42a; a pressing screw 42c for pressing the stem chip 42b against the differential exhaust port 42a; a housing hole 42d that communicates with the differential exhaust port 42a, houses the stem chip 42b formed at the bottom of the differential exhaust port, and screws the pressing screw 42c; and a differential exhaust port extension pipe 42e that communicates with the housing hole 42d and protrudes from the holder main body 12a in the horizontal direction in attachable and detachable state so as to extend the differential exhaust port 42a. This differential exhaust port extension pipe 42e is connected to an exhaust pump, etc. (not shown) air-tightly. After the gas is exhausted from the space between the elastomer seal 41a and the elastomer seal 41b through the differential exhaust port extension pipe 42e, the exhaust unit 42 vacuum-seals so as not to allow the gas to go out of or come into the differential exhaust port 42a by the stem chip 42b.

To the sample mounting part 12b, a sample fixing plate 13 is provided to fasten the outer periphery of the sample 17 from the top side. The sample fixing plate 13 is a plate-like material having a through hole that corresponds to the open window W of the sample holder 12 and the observation position of the sample 17. Its outer shape is larger than the sample 17, and it is fastened to the sample holder 12 by mounting screws, etc. at positions outside the sample 17. The sample 17 is mounted to the sample mounting part 12b so as to block the open window W, and the sample 17 is sealed by the sample fixing plate 13. The sample 17 is thus disposed as a diaphragm dividing between the analysis chamber 11 and the hydrogen cell 12c.

It is only necessary that the outer dimension of the sample 17 is sufficient to block the window area of the open window W. For example, its diameter may be 8 mm and its thickness may be 1 mm. The thickness of the measurement part of the sample 17 disposed as a diaphragm may be approximately the same as the size of the crystal grain of the sample, 100 to 300 µm for example. To the outer peripheral part of the measurement part of the sample 17, a thick part of 500 µm to 2,000 µm may be provided as a part abutting against the window frame area of the open window W.

In the above description, an example where the sample 17 is mounted to the sample mounting part 12b by the sample mounting plate 13 in attachable and detachable state is shown. However, it is also allowed that the sample 17 is fastened to the sample mounting part 12b by welding so as to block the open window W of the sample mounting part 12b. The sample 17 to be welded may be a thin plate made of steel or stainless steel. As the sample 17 to be mounted to the sample mounting part 12b, not only a single sample 17 but also two or more samples 17 are allowed.

The hydrogen gas introduction unit 45 is provided to exhaust the hydrogen cell 12c and introduce hydrogen gas as an observation target gas at a position different from the open window W opening toward the top of the holder main body 12a of the sample holder 12, at the bottom of the hydrogen cell 12c housing the hydrogen absorbing alloy 12d in this embodiment. The hydrogen gas introduction unit 45 comprise: a hydrogen cell exhaust and hydrogen introduction port 45a that is open toward the hydrogen cell 12c and is directly connected to the space within the hydrogen cell 12c; and a stem chip 45b as an opening and closing valve that opens and closes the hydrogen cell exhaust and hydrogen introduction port 45a. The hydrogen cell exhaust and hydrogen introduction port 45a may have an exhaust port and an introduction port at differential positions. However, in this embodiment, they are formed integrally.

The hydrogen gas introduction unit 45 is provided with a pressing screw 45c for pressing the stem chip 45b against the hydrogen cell exhaust and hydrogen introduction port 45a, and a housing hole 45d that communicates with the hydrogen cell exhaust and hydrogen introduction port 45a, houses the stem chip 45b, and screws the pressing screw 45c. At the bottom end of the holder main body 12a, a hydrogen cell evacuation and hydrogen introduction port extension pipe 45e is inserted into the holder main body 12a in attachable and detachable state so as to communicate with the housing hole 45d and extend the hydrogen cell exhaust and hydrogen introduction port 45a. This hydrogen cell evacuation and hydrogen introduction extension pipe 45e is connected air-tightly to an exhaust pump and a hydrogen supply means (not shown) in a state where the switching is allowed as required. When the observation target gas is the one other than hydrogen, the hydrogen cell evacuation and hydrogen introduction port extension pipe 45e serves as an exhaust port and introduction path for exhausting the observation target gas from the cell and introducing the observation target gas into the cell.

The hydrogen cell exhaust and hydrogen introduction port 45a, which communicates with the hydrogen cell 12c, is vacuum-sealed by the stem chip 45b so as not to allow gas to go out and come in, in a state where the sample 17 is mounted to the sample mounting part 12b, the hydrogen cell 12c is exhausted, and hydrogen gas is supplied.

The hydrogen ion detecting unit 20 provided in the analysis chamber 11 comprises: a collecting mechanism 21 for collecting hydrogen ions generated from the surface of the sample 17; an ion energy decomposing unit 22 for removing objects other than hydrogen ions; and an ion detector 23 for detecting hydrogen ions that have passed the ion energy decomposing unit 22.

This hydrogen ion detecting unit 20 detects hydrogen ions generated on the surface of the sample 17 by the ESD method. A two-dimensional image of the hydrogen ions detected by electron beam 16a scanning is called an ESD image or ESD map.

The hydrogen sealed within the hydrogen cell 12c contacts the back surface of the sample 17 from the bottom, is introduced into the sample 17 from this back surface, diffuses within the sample 17, reaches the front surface of the sample 17, and is released. Hydrogen or deuterium permeate from the back side to the front surface of the sample 17, and by emitting the electron beam 16a to the hydrogen that has reached the front surface of the sample 17, hydrogen ions are generated. The generated hydrogen ions are desorbed from the sample 17 by electron stimulated desorption (ESD), and collected by the collecting mechanism 21. Thus, hydrogen ions are detected by the hydrogen ion detecting unit 20.

The collecting mechanism 21, which collects detached ions efficiently, is disposed in the vicinity of the front side of the sample 17. The collecting mechanism 21 shown is made of metal wire mesh, for example, and is a grid-structure lens. The ions of the observation target gas, such as hydrogen ions, collected by the collecting mechanism 21 enter the hydrogen ion detecting unit 20. The ion energy decomposing unit 22 sorts out hydrogen ions and allows them to enter into the ion detector 23.

The ion energy decomposing unit 22 is a metal electrode in a shape of a lid, which prevents the ion detector 23 from directly facing the sample 17. As the ion energy decomposing unit 22, electrodes in a cylindrical, conical, and other shapes can be used. The ion energy decomposing unit 22 applies an appropriate positive voltage to the electrode in a cylindrical shape, introduces only observation target gas ions, hydrogen ions for example, by electric field to the ion detector 23, and removes light and electrons generated by irradiating the sample 17 with the electron beam 16a. As the ion detector 23, Ceratron or a secondary electron multiplier can be used, for example.

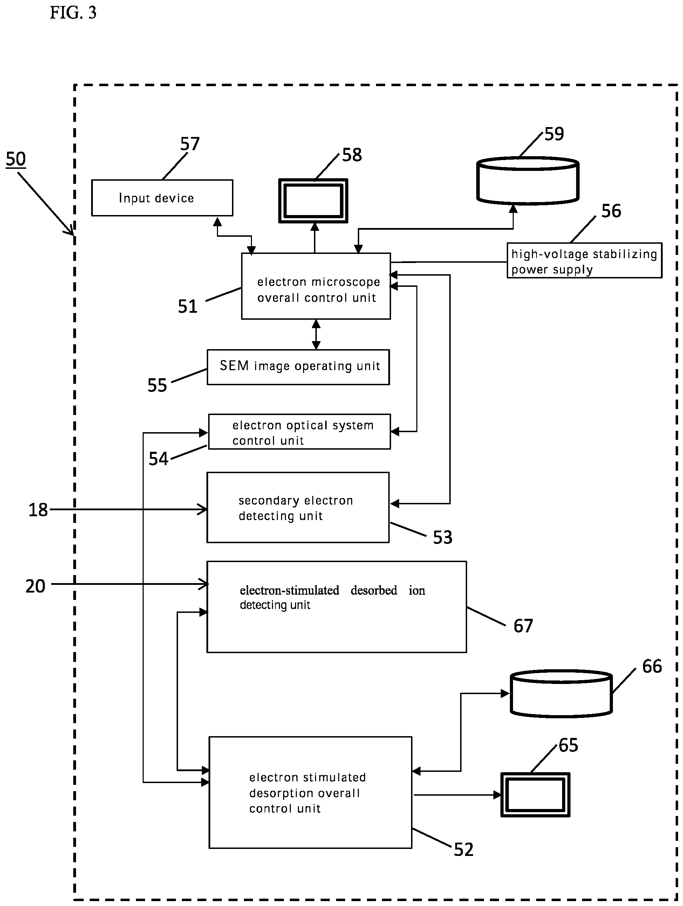

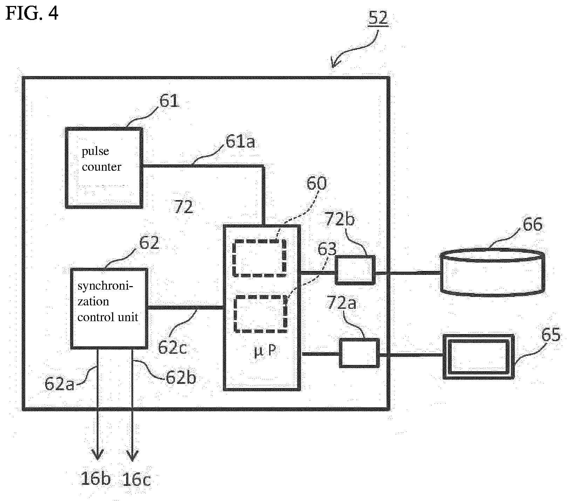

FIG. 3 is a block diagram showing the control unit 50, and FIG. 4 is a block diagram showing the structure of the electron stimulated desorption overall control unit 52. As shown in FIG. 3, the control unit 50 comprises: an electron microscope overall control unit 51 for controlling the scanning electron microscope 15; and an electron stimulated desorption overall control unit 52 for controlling the acquisition of ESD images.

In addition to the electron microscope overall control unit 51, the control unit 50 includes: a secondary electron detecting unit 53, an electron optics system control unit 54, a SEM image operating unit 55, a high-voltage stabilizing power supply 56, an input device 57, a display 58, a memory unit 59, etc., to obtain a scanning electron micrograph (SEM image) of the sample 17. The electron microscope overall control unit 51 controls each of the secondary electron detecting unit 53, the electron optics system control unit 54, the SEM image operating unit 55, the high-voltage stabilizing power supply 56, and the memory unit 59. The output from the secondary electron detector 18 disposed within the analysis chamber 11 is input into the secondary electron detecting unit 53.

As shown in FIG. 4, the electron stimulated desorption overall control unit 52 includes: a two-dimensional multichannel scaler 60; a pulse counter 61; a synchronization control unit 62; a unit for sorting measured signals into two-dimensional surface 63; and a microprocessor 72, etc.

The output from the hydrogen ion detecting unit 20 disposed within the analysis chamber 11 is input into the pulse counter 61 via an electron-stimulated desorbed ion detecting unit 67. To the electron stimulated desorption overall control unit 52, a scanning signal is input from the electron optics system control unit 54, and controlled in synchronization with a SEM image. Furthermore, to the electron stimulated desorption overall control unit 52, a display 65 and a memory unit 66 are connected.

The microprocessor 72 may be a microcomputer such as microcontroller, etc., personal computer, and field-programmable gate array (FPGA).

With this electron stimulated desorption overall control unit 52, the scanning signal input from the electron optics system control unit 54 to the synchronization control unit 62 is output to a first deflection coil 16b of the electron source 16 via the synchronization control unit 62 as a vertical scanning signal 62a.

A horizontal scanning signal 62b from the synchronization control unit 62 is output to a second deflection coil 16c of the electron source 16. The information on the scanning position 62c is output from the synchronization control unit 62 to the microprocessor 72.

A hydrogen ion count signal 61a output from the pulse counter 61 is output to the microprocessor 72 as the count signal of hydrogen ions at each scanning position. The hydrogen ion counts for each sample position counted by the pulse counter 61 may be integrated by obtaining the ESD image within a specified shooting time to acquire the count of hydrogen ions having permeated the sample 17.

The ESD image generated by the microprocessor 72 is output to the display 65 via an input-output interface (I/O) 72a, and also output to the memory unit 66 via an input-output interface (I/O) 72b.

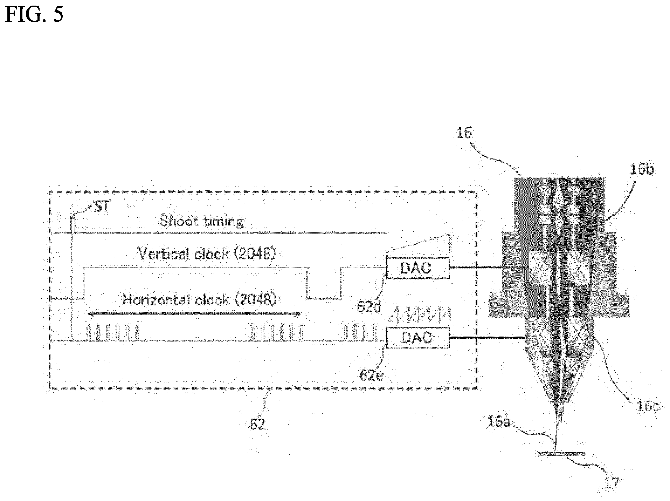

The operation of the electron stimulated desorption overall control unit 52 will be described. FIG. 5 shows the relation between the scanning by the electron source 16 and the two-dimensional measurement of an ESD image. The electron beam 16a generated from the electron source 16 is scanned in both vertical and horizontal directions while passing the first deflection coil 16b and the second deflection coil 16c, and irradiated two-dimensionally to the sample 17.

The clock signal of the vertical scanning signal 62a generated at the synchronization control unit 62 is converted to a sawtooth wave by a digital-analog converter (DAC) 62d, and applied to the first deflection coil 16b of the electron source 16. Similarly, the clock signal of the horizontal scanning signal 62b is converted into a sawtooth wave by a digital-analog converter (DAC) 62e, and applied to the second deflection coil 16c of the electron source 16.

The control is started by one-pulse shoot timing signal (ST signal) so that the vertical scanning signal 62a (vertical clock) generates 2048 pulses in total.

During one pulse width of one-pulse vertical scanning signal 62a, the horizontal pixel signal (horizontal clock) outputs a total of 2048 pulses. Accordingly, two-dimensional scanning having approximately 4,190,000 pixels (2048 rows × 2048 column= 4,194,304) are generated. In other words, the signals counted by the pulse counter 61 can be obtained as the count of hydrogen ions from the ion detector 23 at each scanning position by synchronizing a plurality of counters consisting of ST signal, clock signal for vertical scanning, and clock signal for horizontal scanning.

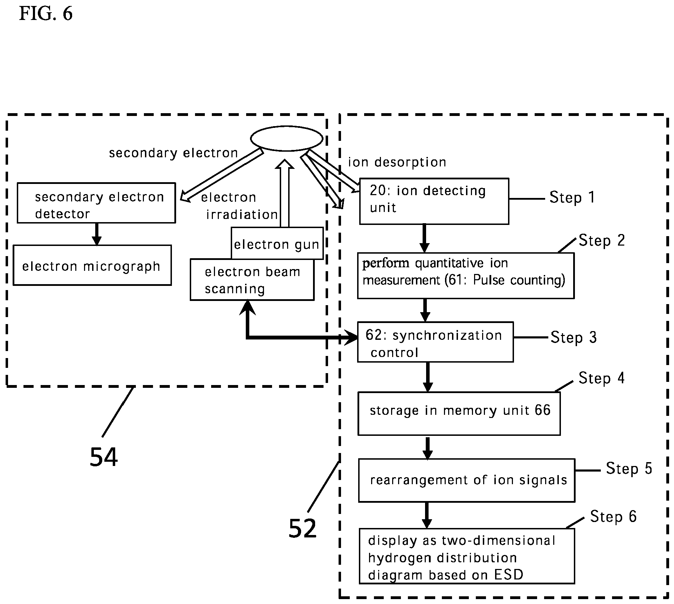

FIG. 6 is a flow chart for measuring a two-dimensional ESD image by scanning. As shown in the chart, the two-dimensional ESD image can be obtained by following the steps as shown below:

| | |
|---|---|
| Step 1: | Ions detached from the surface of the sample 17 are detected by the ion detector 23. |
| Step 2: | The pulse counter 61 performs the quantitative measurement of ions detected by the ion detector 23. |
| Step 3: | Ions at each two-dimensional measurement point of the sample 17 are counted by the synchronization control unit 62 for generating clock signal for vertical scanning and clock signal for horizontal scanning as shown in FIG. 5. |

| | |
|---|---|
| Step 4: | The number of count of ions at each two-dimensional measurement point of the sample 17 measured in step 3 is stored in the memory of the memory unit 66. |
| Step 5: | The ion signals stored in the memory of the memory unit 66 are rearranged as a two-dimensional image based on the clock signal for vertical scanning and the clock signal for horizontal scanning. |
| Step 6: | The ESD image obtained in step 5 is displayed on the display 65 and stored in the memory unit 66 as the image and numerical data. |

The ESD image in the same region as the SEM image can thus be obtained.

The ESD image obtained by following the above steps from 1 to 6 can be executed using the software created in a program creating environment dedicated to measuring instrument control. As such software, Lab VIEW (registered trademark) manufactured by National Instruments Corporation (http://www.ni.com/labview/ja/) can be used. The above-mentioned ESD image can be obtained with the microprocessor 72 by two-dimensional multichannel scaler 60 executed by the program created using LabVIEW.

To measure hydrogen ions originated from hydrogen gas that permeates the sample 17 by using the hydrogen permeation and diffusion path observation device 10 having a sample holder 12 as described above, the following processes must be followed: fabricating a sample 17 outside the device and fastening it to the sample mounting part 12b of the sample holder 12; housing the hydrogen in the hydrogen cell 12c of the sample holder 12; mounting the sample holder 12 to the sample stage 31 and housing it in the analysis chamber 11 of the scanning electron microscope 15 as shown in FIG. 1; and obtaining the SEM and the ESD images.

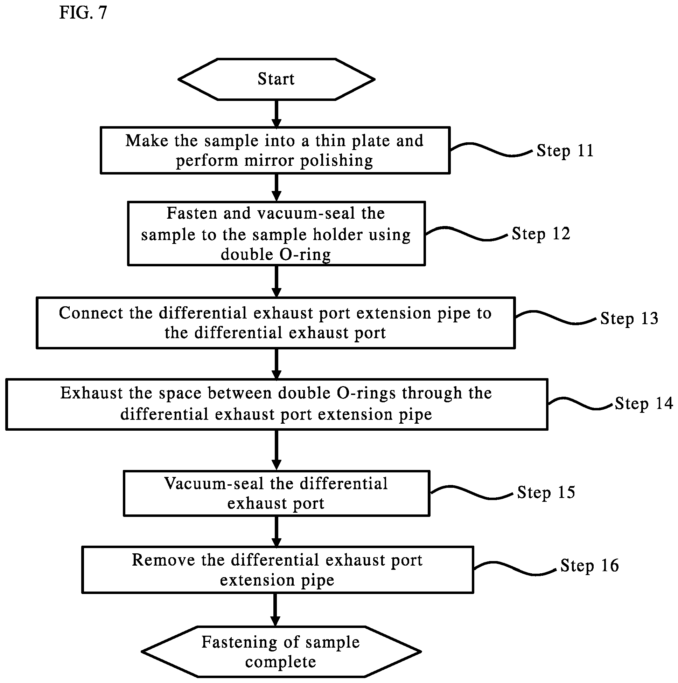

In the process of fastening the sample 17 to the sample holder 12, as shown in FIG. 7, the sample 17 to be observed is fabricated by making it into a thin plate form and mirror polishing is performed in step 11 first. In step 12, the fabricated sample 17 is made to abut against the sample mounting part 12b of the sample holder 12 so as to block the open window W as shown in FIG. 2. To the window frame area of the sample mounting part 12b, a double O-ring made of elastomer seals 41a, 41b is attached, and the back surface of the sample 17 is made to abut against the elastomer seal 41a and the elastomer seal 41b. The sample fastening plate 13 is then mounted, and the sample 17 is pressed down from the top side (front side) for vacuum sealing.

In this state, in step 13, the differential exhaust port extension pipe 42e is connected to the exhaust unit 42 so as to protrude from the holder main body 12a. In step 14, an evacuation means is then connected to the differential exhaust port extension pipe 42e, and exhaust is carried out in a state where the pressing screw 42c is loosened. By evacuating the space between the elastomer seal 41a and the elastomer seal 41b to high vacuum, the sample 17 is appressed against the sample mounting part 12b. Then, in step 15, the vacuum sealing is performed by pressing down the step chip 42b against the seat of the differential exhaust port 42a for blocking using pressing screw 42c. By removing the differential exhaust port extension pipe 42e in step 16, the fastening of the sample 17 is completed.

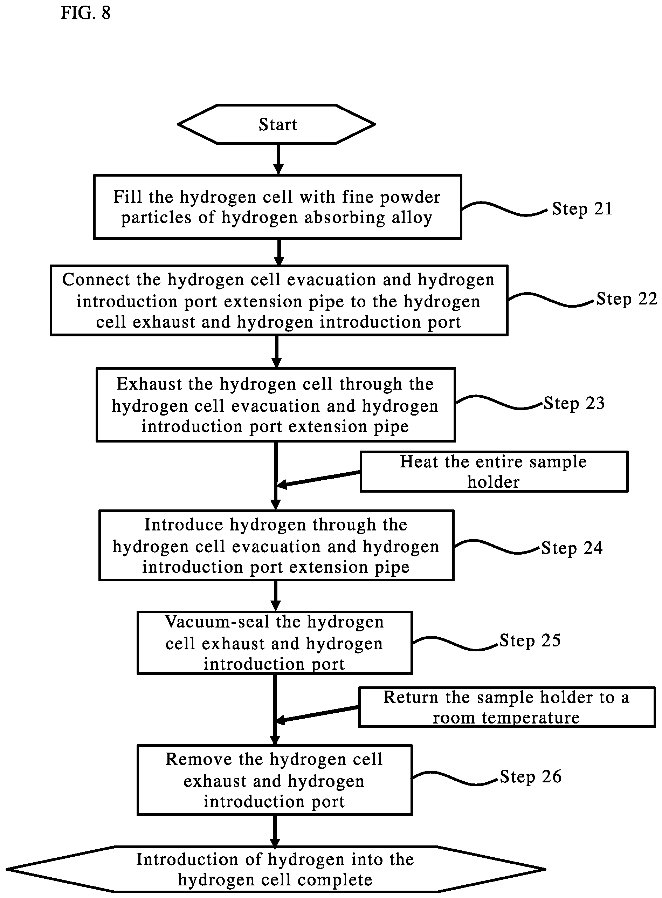

In the process of housing hydrogen in the hydrogen cell 12c of the sample holder 12, as shown in FIG. 8, fine powder particles of hydrogen absorbing alloy are housed in the hydrogen cell 12c in advance in step 21, and then the open window W is blocked by the sample 17 in the process of fastening the sample 17 to the sample holder 12. This may be performed before step 12. In step 22, to the sample holder 12 to which the sample 17 is fastened, the hydrogen cell evacuation and hydrogen introduction port extension pipe 45e is connected. In step 23, an evacuation means is connected to the hydrogen cell evacuation and hydrogen introduction port extension pipe 45e, and by performing evacuation through the hydrogen cell evacuation and hydrogen introduction port extension pipe 45e with the pressing screw 45c loosened, the hydrogen cell 12c is evacuated to a specified degree of vacuum.

In this state, the entire sample holder 12 is preferably heated, and in step 24, the switching is made so that the hydrogen supply means is connected to the hydrogen cell evacuation and hydrogen introduction port extension pipe 45e and hydrogen is supplied. The hydrogen supply means may be a hydrogen gas cylinder, pressure regulator, stop valve, pressure gauge, etc. After the hydrogen gas is supplied, in step 25, the pressing screw 45c is fastened to press down the stem chip 45b against the seat to vacuum-seal the hydrogen cell exhaust and hydrogen introduction port 45a. The entire hydrogen cell 12c is thus blocked securely and hydrogen is maintained within the hydrogen cell 12c by the hydrogen absorbing alloy within the hydrogen cell 12c.

Then, after the sample holder 12 is preferably returned to the room temperature, the hydrogen evacuation and hydrogen introduction port extension pipe 45e is removed in step 26, and thus introduction and housing of hydrogen within the hydrogen cell 12c is completed.

By using the sample holder 12 housing the hydrogen and being sealed entirely, an image acquisition process of the sample is performed. The sample holder 12 is attached to the sample stage 31 so as to be supported by the stage, and disposed within the analysis chamber 11 of the scanning electron microscope 15 as shown in FIG. 1. The analysis chamber 11 is made to be in high vacuum state, and each image is acquired. In the image acquisition process, the control unit 50 allows scanning of electron beam 16a emitted from the electron source 16 to acquire a scanning electron micrograph (SEM image).

Hydrogen is housed within the hydrogen cell 12c of the sample holder 12, and this hydrogen contacts the back side of the sample 17 at the open window W of the sample mounting part 12b. So, atoms that diffuse within the sample and desorb out from the back side to the front surface of the sample 17, hydrogen atoms for example, can be made into hydrogen ions by electron stimulated desorption (ESD) by the electron beam 16a, and an ESD image of the hydrogen ions can be obtained in synchronization with the scanning of the electron beam 16a. In this image acquisition process, it is desirable that the position resolution of the ESD image be 50 nm or lower for comparison with the SEM image.

The internal pressure within the hydrogen cell 12c to be controlled at the time of acquiring the ESD image can be calculated from the temperature at the time of sealing the hydrogen within the hydrogen cell 12c, the sample temperature at the time of measurement, and the equilibrium hydrogen pressure of the hydrogen absorbing alloy. Since the internal pressure also depends on the volume of the hydrogen cell 12c, it is preferable that the control is performed by simplifying the process, by preparing a calibration curve that represents sealing pressure and the temperature at the time of sealing and the sample temperature at the time of measurement in advance, for example.

In this image acquisition process, it is possible to detect hydrogen ions by precisely changing the position of the sample holder 12 using the sample stage 31 as required. It is preferable that the surface of the sample 17 is etched before acquiring a SEM image, and then the SEM image is observed. It is also desirable that the crystal grain boundary be identified from the SEM image, and the identified crystal grain boundary be displayed being overlapped with the SEM and ESD images. The structural information on the discharge position of hydrogen ions can thus be obtained by examining the relation between the crystal grains and the hydrogen ion distribution obtained by the ESD image.

According to the hydrogen permeation and diffusion path observation device 10 and the hydrogen ion observation method of this embodiment, the entire cell 12c can be sealed off in a state where the hydrogen gas is housed within the hydrogen cell 12c, with the hydrogen absorbing alloy 12d placed within the hydrogen cell 12c, and the sample 17 mounted to the sample mounting part 12b. So, if the hydrogen gas is housed in the hydrogen cell 12c and the cell is sealed in advance, the hydrogen gas housed within the hydrogen cell 12c can be made to contact the back surface of the sample 17 when hydrogen ions are detected, and thus it is unnecessary to supply hydrogen gas to the hydrogen cell 12c from outside the analysis chamber 11. It is thus made possible to eliminate the hydrogen gas introduction line to supply the hydrogen gas during observation operation.

Since an advanced observation atmosphere can be maintained around the sample 17 and the sample holder 12 within the analysis chamber 11, the operation of the sample holder 12 within the analysis chamber 11 cannot be inhibited by the hydrogen gas introduction line. It is also possible to independently rotate and transfer the sample holder 12 to which the sample 17 is mounted within the analysis chamber 11. For example, it is possible to observe the sample 17 while rotating it.

According to the present invention, it is possible to combine with the electron backscatter diffraction method and the reflection high-energy electron diffraction method, which are microscopic structural analysis methods whose positional relation between the sample and the detecting device is different from that of the operando hydrogen microscope, or with a plurality of measurement means whose detecting devices cannot be placed in the space within the detector. So, the performance to study various hydrogen gas behaviors, such as finding the diffusion coefficient of the hydrogen gas within the sample 17, will be improved.

In this embodiment, since the sample stage 31 is provided to support the sample holder 12 in attachable and detachable state and operate the holder precisely within the analysis chamber 11, it is possible to house the hydrogen gas outside the analysis chamber 11 and mount the sample 17 to the sample mounting part 12b in advance, and then make the sample stage 31 support the sample holder 12 and precisely operate the sample 17 for observation, which facilitates observation of hydrogen gas behavior.

In this embodiment, since the hydrogen absorbing alloy 12d of the observation target gas is housed within the hydrogen cell 12c, a large amount of hydrogen gas can be housed in the hydrogen cell 12c stably and safely.

In this embodiment, the sample mounting part 12b is provided with the window frame area, against which the sample 17 can abut air-tightly, around the periphery of the open window W, and the sample mounting part 12b has the elastomer seal 41a surrounding the open window W at the window frame area, the elastomer seal 41b surrounding the elastomer seal 41a, the differential exhaust port 42a for exhausting the space between the elastomer seal 41a and the elastomer seal 41b, and the stem chip 42b for opening and closing the differential exhaust port 42a. So, if the stem chip 42b is closed in a state where the sample 17 is made to abut against the sample mounting part 12b so as to be appressed to the elastomer seal 41a and the elastomer seal 41b, and the space between the elastomer seal 41a and the elastomer seal 41b is sufficiently exhausted through the differential exhaust port 42a, the hydrogen cell 12c is blocked even if the hydrogen gas directly contacts the back surface of the sample 17. Even if the differential pressure between the analysis chamber 11 and inside the hydrogen cell 12c is large, the advanced observation atmosphere within the analysis chamber 11 can be maintained without fail.

In this embodiment, since the hydrogen cell exhaust and hydrogen introduction port 45a for exhausting the hydrogen cell 12c and introducing hydrogen gas and the stem chip 45b for opening and closing the hydrogen cell exhaust and hydrogen introduction port 45a are provided, the hydrogen cell 12c can easily be filled with a sufficient amount of hydrogen gas of sufficient quality after the sample 17 is mounted to the sample mounting part 12b in a state where the open window W is blocked.

With the exhaust unit 42 in this embodiment, since the differential exhaust port extension pipe 42e is provided, protruding from the sample holder 12 in attachable and detachable state, the external exhaust means can be easily connected to the differential exhaust port extension pipe 42e, and also if the differential exhaust port extension pipe 42e is removed when observation is made with the sample holder 12 housed in the analysis chamber 11, the observation operation cannot be inhibited, which is very convenient. The hydrogen cell evacuation and hydrogen introduction port extension pipe 45e is also provided, protruding from the sample holder 12 in attachable and detachable state, which is also very convenient.

The above-mentioned embodiment can be modified as required within the scope of the present invention.

The above embodiment was described, taking the example of the hydrogen permeation and diffusion path observation device 10. However, the embodiment is not limited to the described application only. It is also applicable to detect the position of a defect of the sample 17, for example, and also by repetitively observing the same sample 17, the change in the behavior of observation target gas such as diffusion or permeation can be measured.

In the above embodiment, hydrogen was chosen as the observation target gas. However, observation target gas is not limited to hydrogen but other gasses can also be observed. In that case, observation can be made by using the same sample holder 12 as the above example and housing the target gas in the cell 12c. It is also allowed to place an absorbing material within the cell 12c to house the target gas. For example, if the observation target gas is water molecules, a water-absorbing polymeric material may be used as the absorbing material.

### Reference Signs List

- 10:: Hydrogen permeation and diffusion path observation device
- 11:: Analysis chamber
- 12:: Sample holder
- 12a:: Holder main body
- 12b:: Sample mounting part
- 12c:: Hydrogen cell
- 12d:: Hydrogen absorbing alloy
- 13:: Sample fixing plate
- 15:: Scanning electron microscope
- 16:: Electron source
- 16a:: Electron beam
- 16b:: First deflection coil
- 16c:: Second deflection coil
- 17:: Sample
- 18:: Secondary electron detector
- 20:: Hydrogen ion detecting unit (observation target ion supply unit)
- 21:: Collecting mechanism
- 22:: Ion energy decomposing unit
- 23:: Ion detector
- 31:: Sample stage
- 33:: Sample temperature measuring section
- 35:: Mass analyzer
- 36:: Auger electron spectroscopy analyzer
- 37:: Evacuation unit
- 40:: Sealing part
- 41a:: Inner elastomer seal
- 41b:: Outer elastomer seal
- 42:: Exhaust unit
- 42a:: Differential exhaust port
- 42b, 45b:: Stem chip (on-off valve)
- 42c, 45c:: Pressing screw
- 42d, 45d:: Housing hole
- 42e:: Differential exhaust port extension pipe
- 45:: Hydrogen gas introduction unit
- 45a:: Hydrogen cell exhaust and hydrogen introduction port
- 45e:: Hydrogen cell evacuation and hydrogen introduction port extension pipe
- 50:: Control unit
- 51:: Electron microscope overall control unit
- 52:: Electron stimulated desorption overall control unit
- 53:: Secondary electron detecting unit
- 54:: Electron optics system control unit
- 55:: SEM image operating unit
- 56:: High voltage stabilizing power supply
- 57:: Input device
- 58, 65:: Display
- 59, 66:: Memory unit
- 60:: Two-dimensional multichannel scaler
- 61:: Pulse counter
- 61a:: Hydrogen ion count signal
- 62:: Synchronization control unit
- 62a:: Vertical scanning signal
- 62b:: Horizontal scanning signal
- 62c:: Information on scanning position
- 62d, 62e:: Digital-analog converter
- 63:: Unit for sorting measured signals to two-dimensional surface
- 67:: Electron-stimulated desorbed ion detecting unit
- 72:: Microprocessor
- 72a, 72b:: Input-output interface
- W:: Open window

## Claims

1. An observation device for observation target gas, comprising:
a scanning electron microscope for detecting secondary electrons generated by emitting an electron beam to a sample within an analysis chamber;
a sample holder having a cell for housing the observation target gas, an open window of the cell, and a sample mounting part to which the sample can be mounted in a state blocking the open window; and
an observation target ion detecting unit for detecting observation target ions derived from the observation target gas generated by the electron beam after emitting the electron beam to the front surface of the sample in a state where the observation target gas in the cell contacts the back surface of the sample,
wherein the entire cell can be sealed in a state where the observation target gas is housed in the cell and the sample is mounted to the sample mounting part of the sample holder.

2. The observation device for observation target gas as set forth in claim 1, wherein an absorbing material of the observation target gas is housed in the cell.

3. The observation device for observation target gas as set forth in claim 1 or 2, wherein the sample mounting part has a window frame area around the open window against which the sample can abut air-tightly, and the window frame area comprises: an internal seal surrounding the open window; an outer seal surrounding the inner seal; an exhaust port for exhausting the space between the inner seal and the outer seal; and a valve for opening and closing the exhaust port.

4. The observation device for observation target gas as set forth in claim 3, wherein a differential exhaust port extension pipe is installed in the sample holder, communicating with the exhaust port, in an attachable and detachable state.

5. The observation device for observation target gas as set forth in any one of claims 1 to 3, wherein the sample holder comprises: an exhaust port and introduction path for exhausting the cell and introducing the observation target gas at a position different from the open window; and a valve for opening and closing the exhaust port and introduction path.

6. The observation device for observation target gas as set forth in claim 5, wherein the exhaust port and the introduction path is installed in a state attachable to and detachable from the sample holder.

7. The observation device for observation target gas as set forth in claim 1, wherein the sample holder is attached to a sample stage in a state removable to outside the analysis chamber.

8. The observation device for observation target gas as set forth in claim 7, wherein the sample stage is installed within the analysis chamber in an insertable and removable state.

9. The observation device for observation target gas as set forth in claim 8, wherein the sample stage comprises: a rotating mechanism; a temperature control; and an ion focusing mechanism, and the sample stage is configured to heat the sample.

10. A method of observing target ions by using the observation device for observation target gas as set forth in any one of claims 1 to 9, the method of observing target ions comprising the steps of:
mounting the sample to the sample mounting part to block the open window;
housing the observation target gas in the cell,
placing the entire sample holder within the analysis chamber with the entire cell sealed; and
detecting the observation target ions derived from the observation target gas generated by emitting the electron beam to the front surface of the sample in a state where the observation target gas contacts the back surface of the sample.

11. The method of observing target ions as set forth in claim 10, wherein the observation target ions are detected by precisely changing the position of the sample holder within the analysis chamber.

12. The method of observing target ions as set forth in claim 10 or 11, wherein a material for absorbing the observation target gas and the observation target gas are housed in the cell.

13. The method of observing target ions as set forth in any one of claims 10 to 12, wherein
mounting the sample to the sample mounting part in a state where the sample is appressed against a window frame area surrounding the open window, blocking the open window;
exhausting the cell and introducing the observation target gas to the cell at a position different from the open window; and
placing the entire sample holder within the analysis chamber after the entire cell is sealed.

14. A sample holder, comprising:
a holder main body that can be housed within an analysis chamber of a scanning electron microscope for detecting secondary electrons by emitting an electron beam;
a cell for housing an observation target gas provided within the holder;
a sample mounting part to which a sample can be mounted; and
an open window of the cell provided at the sample mounting part, wherein
by mounting the sample to the sample mounting part, blocking the open window, the cell is sealed in a state where the observation target gas contacts the back surface of the sample.
